# EUROPÄISCHE PATENTSCHRIFT

(11) **EP 1 414 315 B1**
(45) Veröffentlichungstag und Bekanntmachung des Hinweises auf die Patenterteilung: **07.03.2007**
(21) Anmeldenummer: 02758426.7
(22) Anmeldetag: 02.08.2002
(51) Int. Cl.: A23L 1/30, A23L 1/302, A23L 1/303, A23L 1/304, A23L 1/305, A61K 31/355, A61K 31/455, A61K 31/375, A61K 36/00, A61K 36/02

(54) **KOMPLEXES ANTIOXIDANTIENPRÄPARAT**
COMPLEX ANTIOXIDANT PREPARATION
PREPARATION ANTIOXYDANTE COMPLEXE

(30) Priorität: 02.08.2001 DE 10137827
(43) Veröffentlichungstag der Anmeldung: 06.05.2004
(73) Patentinhaber: Marcinowski, Peter, 88709 Meersburg (DE)
(72) Erfinder: Marcinowski, Peter, 88709 Meersburg (DE)
(74) Vertreter: Kramer - Barske - Schmidtchen
(86) Internationale Anmeldenummer: PCT/EP2002/008649
(87) Internationale Veröffentlichungsnummer: WO 2003/011054

(56) Entgegenhaltungen:
- EP-A- 0 127 012
- WO-A-01/10252
- WO-A-94/05280
- WO-A-99/15035
- WO-A-99/61038
- DE-A- 19 930 221
- FR-A- 2 803 490
- US-A- 5 083 578

## Beschreibung

Die Erfindung betrifft ein komplexes Antioxidantienpräparat, eine Verwendung des Präparates, eine oral einnehmbare Kapsel, eine Kautablette sowie eine Schachtel mit den Abmessungen einer Zigarettenschachtel für Automaten.

Das Rauchen von Tabak gehört zu den ältesten Genussmitteln der Menschheit. Trotz aller Hinweise auf die schädlichen Wirkungen, insbesondere des Zigarettenrauchens, konnte der Konsum nicht wesentlich eingeschränkt werden. Derzeit rauchen weltweit etwa 70% der männlichen und 35% der weiblichen Bevölkerung. Die Folgeschäden durch Rauchen werden allein in der Bundesrepublik auf mindestens jährlich 30 Milliarden DM geschätzt.

Rauchen, insbesondere das in ihm enthaltene Nikotin, steigert kurzfristig das Wohlbefinden, indem das Gehirn kurzzeitig wacher wird und Spannungen sowie Ängste gedämpft werden. Die kurzfristig angenehmen Momente des Rauchens überdecken die negativen, sich langsam einschleichenden Zerstörungen des Organismus. Diese negativen Wirkungen gehen sowohl vom aktiven Rauchen aus, d.h., dem Rauch, der unmittelbar durch Ziehen an der brennenden Zigarette in die Lunge eingesaugt wird, als auch vom passiven Rauchen, indem der Qualm einer Zigarette eingeatmet wird. Dabei ist der "passive Rauch" wegen der in ihm enthaltenen, noch nicht aufoxidierten Bestandteile zum Teil noch schädlicher als der "aktive Rauch".

Bis heute sind 300 Schadstoffe und etwa 4000 chemische Verbindungen im Tabakrauch bekannt. Die wichtigsten Schadstoffe sind folgende:
a) Nikotin
b) Kohlenmonoxid
c) Reizgase und Aldehyde
d) Stickstoffverbindungen
e) Aromatische und aliphatische Kohlenwasserstoffe.

Im folgenden werden schädlichen Wirkungen der vorgenannten Schadstoffgruppen kurz zusammengefasst:
a) Nikotin
   Nikotin ist ein starkes, auf das Nervensystem wirkendes Gift, das süchtig machen kann. Nikotinvergiftungen gehen mit Brechreiz, Zittern und Krämpfen einher. Die Giftwirkung einer einzigen Zigarette kann ein kleines Kind töten und würde auch bei einem Erwachsenen zu schweren Vergiftungserscheinungen führen, sofern er die Zigarette isst, anstatt zu rauchen. Nikotin wird in der Zigarettenglut jedoch zu großen Teilen vernichtet und nur ein kleiner Teil gelangt in die Lunge und somit in die Blutbahn. Der Effekt auf das Herz-Kreislauf-System geht in erster Linie über das Adrenalin. Dieses Hormon verursacht höhere Pulsfrequenz und höheren Blutdruck, indem die Hautgefäße verengt und die Muskelgefäße erweitert werden.
b) Kohlenmonoxid
   Kohlenmonoxid gehört zu den Atemgiften und bildet sich nicht nur in Tabakrauch, sondern auch in schlecht belüfteten engen Straßen und Tunneln, schlecht ziehenden Öfen, usw.. Das Blut des Rauchers enthält 10-15% Kohlenmonoxid. Kohlenmonoxid verbindet sich mit dem roten Blutfarbstoff 200 bis 300 mal leichter als Sauerstoff, deshalb leiden Raucher ständig unter einem relativen Sauerstoffmangel. In Verbindung mit der Adrenalin erzeugenden Wirkung des Nikotins kommt es zu einem zusätzlichen Sauerstoffdefizit, auf den der Organismus mit Bildung weiterer Blutkörperchen reagiert. Dadurch wiederum wird das Blut dickflüssiger und die Thrombosegefahr erhöht sich. Nach neueren Erkenntnissen ist das erhöhte Risiko des Rauchens an Herz-Kreislauf-Schäden zu erkranken, hauptsächlich auf den relativen Sauerstoffmangel im Blut zurück zu führen.
c) Reizgase und Aldehyde
   In der Verbrennung organischer Verbindungen entstehen Aldehyde, die als potente Radikalbildner gelten und zudem sogenannte "Crosslinker" sind. Crosslinker verfügen in ihrem Molekülverbund über zwei reaktionsfreudige Bestandteile, die nach dem Zufallsprinzip mit x-beliebigen Substanzen Verbindungen eingehen und damit regelrechte Flechtwerke bilden, die wiederum nachhaltig zerstörerisch auf unseren Organismus wirken.
d) Stickstoffverbindungen
   Raucher belasten sich mit im Rauch vorhandenen Nitroverbindungen. Nitrate sind an sich nicht giftig und werden fast völlig durch Nieren und Darm wieder ausgeschieden. Sie können jedoch durch Mikroben im menschlichen Darm leicht in Nitrite umgewandelt werden. Nitrite wiederum bilden einen denaturierten roten Blutfarbstoff, das Methämoglobin, der für denSauerstofftransport nicht mehr geeignet ist. In den vergangenen Jahren zunehmend beobachtete Dickdarmkrebs-Erkrankungen werden auf die Umwandlung der an sich harmlosen Stickstoffverbindungen unter Mitwirkung derDarmbakterien zurückgeführt.
e) Aromatische und aliphatische Kohlenwasserstoffe
   Diese bei der Verbrennung von Tabak entstehenden Substanzen sind starke Radikalbildner. Die krebserzeugende und mutagene Wirkung beruht sehr wahrscheinlich auf Schädigungen der Erbsubstanz (DNA) und der Membranen um bzw. in den Zellen. Das im Tabakrauch enthaltene Benzpyren ist ein bekannter Vertreter dieser Gruppe. Am meisten geschädigt werden jene Gewebe, die in direktem Kontakt mit dem Rauch stehen, also Mundschleimhaut, Kehlkopf, Bronchien und Lunge. Fest steht, dass der Organismus des Rauchers unter schwerem oxidativem Stress steht und im Vergleich zum Nichtraucher erheblich früher altert.

Ebenso, wie es in der Vergangenheit nicht gelungen ist, trotz des Wissens über die gesundheitsschädlichen Wirkungen des Rauchens den Zigarettenkonsum wesentlichzurückzudrän- gen, wird dies in der Zukunft nicht gelingen, da von den kurzfristig angenehmen Momenten des Rauchens immer eine anziehende Wirkung ausgehen wird. Die Emotionen - das ist uns Menschen eigensiegen nur allzu gerne über die Ratio, die Vernunft.

WO 01/0252 beschreibt ein Antioxidantienpräparat enthaltend Anthocyanin, Carotenoid, Isoflavon, Vitamin E un Cystein, das geeignet ist, die schädlichen Wirkungen des Rauchens zu vermindern.

Aus der WO 99/150305 ist ein Antioxidatienprodukt bekannt, das einer Zigarette, Zigarre oder Pfeife unmittelbar beigegeben werden kann. Das Produkt kann dem Tabak selbst zugegeben werden, einem Filter zum Filtern des Tabekrauches oder dem Papier bzw. der Ummantelung, die den Tabak umgibt. Das Produkt ist in der Lage, Schädigungen des oro-pharyngealen Hohlraums, des Atemtrakts und der Lungen zu vermindern, die durch freie Radikale ausgelöst werden. Das Produkt enthält L-Glutation und eine Selenquelle, beispielsweise Selenometionin.

Der Erfindung liegt die Aufgabe zugrunde, die schädlichen Wirkungen des Rauchens zu vermindem.

Eine Lösung der Aufgabe wird mit einem komplexen Antioxidantienprodukt gemäss dem Anspruch 1 erzielt. Das erfindungsgemässe, komplex aus mehreren Antioxidantien zusammengesetzte Präparat mit hohen Anteilen pflanzlicher Wirkstoffe, das vorteilhafterweise oral eingenommen wird, mindert die schädliche Wirkung des Tabakrauches.

Die Erfindung macht sich die Erkenntnis zunutze, dass den schädlichen Wirkungen freier Radikale, die durch Wirkungen des Zigarettenrauches im Körper freigesetzt werden (Verbindungen mit Elektronendefizit infolge einer gespaltenen Atombindung), dadurch entgegen gewirkt werden kann, dass diese durch sogenannte Antioxidantien, d. h., Elektronendonatoren, zumindest teilweise unschädlich gemacht werden können. Solche, als Radikalfänger wirkende Antioxidantien bleiben nach Abgabe eines Elektrons, d.h., nach ihrer eigenen Oxidation, entweder stabil oder können vom Organismus in Verbindung mit weiteren Antioxidantien neutralisiert, regeneriert oder ausgeschieden werden. Das bedeutet, dass solche Antioxidantien bei einer erhöhten Anzahl zu beseitigender freier Radikale in erhöhtem Ausmaß vorhanden sein müssen.

Im folgenden werden die Wirkungen der vier, in dem Anspruch 1 aufgeführten Wirkstoffgruppen, die als Antioxidantien wirken, erläutert:

### A. Die wasserlöslichen Vitamine

Vitamin C ist das wichtigste und entscheidende Antioxidans im wässrigen System unseres Körpers. Vitamin C reagiert vor allem mit toxischen Sauerstoffradikalen (Superoxidation, Hydroxylradikale, Peroxidradikale). Vitamin C fängt diese Radikale ab und verhindert ihr Eindringen in das Lipidsystem. Freie Radikale, die diese Schranke überwinden, können in die Lipidphase vordringen und dort durch Oxidation der Lipide (Lipidperoxidation) Schäden verursachen. Daneben unterstützt Vitamin C die antioxidative Wirkung von Vitamin E; im Darm verhindert es die Bildung von Nitrosaminen.

Vitamin B3 (Niacin) gehört zu den Vitaminen der B-Gruppe. Sie schützen Zellen und Gewebe gegen viele Umwelt- und Genussgifte. Niacin sichert die Funktion des Nervensystems, repariert geschädigte Erbinformationen und ist an der Erhaltung der Sauerstoffkapazität im Blut beteiligt. Für Raucher ist die gegenteilige Wirkung zum Nikotin interessant: Es erweitert die durch Nikotin verengten Gefäße und ist beim Abräumen der durch das Rauchen verursachten Fettablagerungen beteiligt. Niacin wird deswegen auch bei Arteriosklerose empfohlen.

Beta-Carotin ist ein starkes Antioxidans und neutralisiert Sauerstoffradikale. Darüber hinaus fängt Beta-Carotin schädliche Radikale ab und schützt so LDL-Cholesterin vor der Oxidation.

Vitamin E als wichtigstes fettlösliches Antioxidans verhindert vor allem die Oxidation der ungesättigten Fettsäuren (Lipidperoxidation). Vitamin E wird durch Vitamin C teilweise im Organismus wieder regeneriert und ist deshalb auf das Vorhandensein von Vitamin C angewiesen.

### B. Die fettlöslichen Vitamine

Da die Antioxidantien bei erhöhtem oxidativem Stress verbraucht werden, ist eine tägliche Substitution erforderlich.

### C. Bioaktive Pflanzenstoffe

Reichhaltige Quelle für Citrus-Bioflavonoid-Komplexe sind die Schale und das Fruchtfleisch von Citronen. Bioflavonoide wirken als Antioxidantien. Die Antioxidantien erhöhen die Aufnahme von Vitamin C und schützen die Wände von Blutgefäßen und Kapillaren.

Die Wirkung des Traubenschalenextrakts beruht auf der Fähigkeit der Flavonoide, Elektronen abzugeben, sowie freies Eisen zu binden. In neueren Studien konnte gezeigt werden, dass Flavonoide LDL-Partikel nur so lange vor Oxidation schützen können, wie auch Vitamin E im Medium vorhanden war.

### D. Bioorganische Moleküle

L-Cystin gehört zu den Schwefel-haltigen Verbindungen, die sich durch eine besondere antioxidative Wirkung auszeichnen. Schwefel-haltige Nährstoffverbindungen schützen besonders Organe mit hoher Zellteilungsrate und fördern den Zellaufbau. Über reduziertes Glutathion wird mit Hilfe der Glutathionperoxidase, die zudem noch Selen benötigt, um das den Respirationstrakt auskleidenden Epithelial-Lining-Fluid zu erhalten; zudem wird das so wichtige Flimmerepithel in der Lunge dadurch stabilisiert.

Folsäure (Vitamin M) ist eine Verbindung aus Pteridin, Para-Aminobenzoesäure und Glutaminsäure. Sie wird auch als Pteroylglutaminsäure bezeichnet. Folsäuremangel und Vitamin B-Defizite führen zu erhöhten Homocysteinwerten im Blut. Homocystein wiederum schädigt die Endozelzelle der arteriellen Gefäße und erhöht die Bindung von Lipoprotein A an Fibrin, so dass das Blut in der Folge krankhaft verdickt wird. Folsäure ist besonders für jene Zellen wichtig, die eine hohe Teilungsrate aufweisen, so auch in den Schleimhäuten.

Meeresalgenpulver enthält zahlreiche Mineralien und Spurenelemente in einer für den Körper verfügbaren Form auf pflanzlicher Basis. Die oft nur in geringsten Dosierungen notwendigen Spurenelement-Substitution können bei erhöhtem oxidativen Stress auf pflanzlicher Basis komplex dargeboten werden.

Selen als Bestandteil des Gluathion-Enzymsystems (Glutathionperoxidase) ist im Gesamtgefüge der antioxidativen Schutzsysteme ein sehr wichtiger Partner, werden durch ihn doch erst einige Enzymsysteme überhaupt wirksam.

Zink ist Bestandteil der zinkhaltigen Superoxiddismutase (SOD), eines wichtigen antioxidativen, zellschützenden Enzyms. Im Immunsystem ist Zink lebensnotwendig für die Ausbildung der Lymphocyten, wie auch bei der Antikörperproduktion der natürlichen Killerzellen. Viele Schüsselelemente der Nucleinsäuresynthese, des Zellkerns und der zellulären Proteinsynthese sind Zink abhängig.

Die Komplexität des erfindungsgemäßen Antioxidantienpräparates, d.h., die Kombination der in ihm enthaltenen Wirkstoffgruppen, ist deshalb vorteilhaft, weil diese Wirkstoffgruppen in Kombination synergistisch wirken. Durch die Kombination wird eine Wirkung erzielt, die besser ist, als einer Addition der Einzelwirkungen entsprechend.

Das erfindungsgemäße komplexe Antioxidantienpräparat eignet sich zur Lieferung von ganz allgemein für den Stoffwechsel notwendigen Radikalenfängern. Besonders gut eignet sich das erfindungsgemäße Antioxidantienpräparat zur Linderung schädlicher Wirkungen des Rauchens von Tabakprodukten. Nach aktuellem Kenntnisstand empfiehlt sich eine Gabe von etwa 10-25mg Vitamin C (und daraus abgeleitet die entsprechende Menge an Gesamtwirkstoff des erfindungsgemäßen Präparates entsprechend dessen Zusammensetzung) je Zigarette. Der obere Dosierungswert gilt für schwere Zigaretten mit hohem Gehalt an Nikotin und anderen schädlichen Bestandteilen.

Passiv-Raucher, d.h., nicht selbst rauchende Personen, in einer Umgebung, in der stark geraucht wird, verwenden zweckmäßigerweise eine Dosierung ähnlicher Größenordnung.

Der Anspruch 2 kennzeichnet eine vorteilhafte Zusammensetzung des erfindungsgemäßen Präparates absolut und in Gew.-%, wobei die Bestandteile durchaus um jeweils +/-20% ihres Wertes schwanken können.

Vorteilhafterweise wird das erfindungsgemäße Antioxidantienpräparat in Form oral einnehmbaren Kapseln dargeboten. Solche Kapseln sind als herunter zu schluckende Kapseln konzipiert und enthalten eine Hülle, die beispielsweise aus Kartoffelstärke besteht.

Vorteilhafterweise kann das erfindungsgemäße Antioxidantienpräparat auch in Form einer Kautablette dargeboten werden, die als Grundsubstanz Dextrose mit gegebenenfalls zusätzlichen Ballaststoffen und Aromastoffen enthalten kann.

Die Kautablette kann zusätzlich eine Atem-Refresher enthalten, um den Rauchgeruch in der ausgeatmeten Luft zu überdecken.

Eine Kapsel oder eine Kautablette enthält beispielsweise 60mg Vitamin C und die weiteren Bestandteile entsprechend der im Anspruch 7 angegebenen Zusammensetzung.

Vorteilhaft ist es, das erfindungsgemäße Antioxidantienpräparat in Form von Kautabletten oder Kapseln oder sonstwie darzubieten, die in einer Schachtel mit den Abmessungen einer Zigarettenschachtel verpackt sind, so dass die Schachtel mit dem erfindungsgemäßen Antioxidantienpräparat zusammen mit Zigaretten in Zigarettenautomaten angeboten werden kann. Die Empfehlung, je wie viele Zigaretten vorteilhafterweise eine Kapsel bzw. eine Kautablette genommen wird oder wie häufig eine Kapsel oder Tablette in stark rauchhaltiger Umgebung eingenommen wird, wird vorteilhafterweise auf der Schachtel angegeben, so dass der Kunde **die Einnahme zweckmäßig dosieren kann.**

Die Herstellung des erfindungsgemäßen Antioxidantienpräparates, beispielsweise mit der Zusammensetzung gemäß dem Anspruch 2, ist wie folgt:

Die angegebenen Rohstoffe sind im Handel in hoher Reinheit und guter Qualität, beispielsweise DAB- oder EP-Qualität, erhältlich. Die Rohstoffe werden in den angegebenen relativen Mengen erforderlichenfalls zerkleinert und dann sorgfältig vermischt. Aus der Mischung werden in an sich bekannter Weise, gegebenenfalls nach Zugabe weiterer Stoffe, wie Trägermaterialien, Ballaststoffen, Geschmacksstoffen, usw., Kapseln, Kautabletten, Verzehrriegel, uws., hergestellt. Zu schluckende Kapseln enthalten vorteilhafterweise nur die in den Ansprüchen angegebenen Wirkstoffe. Eine Kautablette enthält beispielsweise etwa 300mg Maltodextrin je 700mg Wirksubstanz.

In einem, in seinem Aufbau an sich bekannten, Produktautomaten, bei dem ein konventioneller Zigarettenpackungsschacht mit erfindungsgemäßen Schachteln gefüllt ist, kann das erfindungsgemäße Produkt, bequem erreichbar, dargeboten werden.

## Patentansprüche

1. Komplexes Antioxidantienpräparat, enthaltend
Niacin (B3), Vitamin C, Beta-Carotin, Vitamin E, Citrus-Bioflavonoide, Traubenschalenextrakt, L-Cystin, Folsäure, Meeresalgenpulver und ein enzymatisches Schutzsystem mit einer Zink-, Selen-, Kupfer- und / oder Mangan-Verbindung.

2. Antioxidantienpräparat, enthaltend folgende relative Zusammensetzung (Gewichtsprozente), wobei die Prozentzahlen jeweils um ± 20 % ihres Wertes schwanken können:
| Rohstoff | Zusammensetzung Gew.-% |
|---|---|
| Vitamin C (Ascorbinsäure) | 29,34 |
| Beta-Carotin 10% | 7,33 |
| Vitamin E-acetat 50 % | 13,13 |
| Folsäure | 0,54 |
| Nicotinsäureamid | 4,40 |
| L-Cystin | 12,22 |
| Selenhefe 0,2 % | 6,11 |
| Zinkhefe 10% | 0,05 |
| Traubenschalenextrakt | 2,44 |
| Citrusbioflavonoid | 2,44 |
| Seealgenmehl | 19,56 |
| Fruchtpulver Hagebutte | 2,44 |

3. Oral einnehmbare Kapsel, enthaltend ein komplexes Antioxidantienpräparat nach Anspruch 1 oder 2.

4. Kautablette, enthaltend ein Antioxidantienpräparat nach Anspruch 1 oder 2.

5. Kautablette, nach Anspruch 4, enthaltend einen Atem-Refresher.

6. Schachtel mit Abmessungen einer Zigarettenschachtel für Automaten, enthaltend Produkte nach Anspruch 3 bis 5.

7. Produktausgabeautomat, enthaltend mehrere getrennt anwählbare Warenschächte mit darin gestapelten Schachteln, wobei zumindest einer der Warenschächte Zigarettenschachteln und zumindest ein weiterer der Warenschächte Schachteln nach Anspruch 6 enthält.

## Claims

1. Complex anti-oxidant preparation comprising:
niacin (B3), vitamin C, beta-carotene, vitamin E, citrus bioflavonoids, extract of grape skin, L-cystine, folic acid, seaweed powder and an enzymatic protective system having a zinc-, selenium-, copper- and/or manganese-compound.

2. Anti-oxidant preparation comprising the following relative composition (weight percent), wherein the percent amounts can each vary by ±20 % of its value:
| Raw material | Composition wt.-% |
|---|---|
| Vitamin C (ascorbic acid) | 29.34 |
| Beta-carotene 10% | 7.33 |
| Vitamin E-acetate 50% | 13.13 |
| Folic acid | 0.54 |
| Nicotine acid amide | 4.40 |
| L-cystine | 12.22 |
| Selenium-yeast 0.2% | 6.11 |
| Zinc-yeast 10% | 0.05 |
| Extract of grape skin | 2.44 |
| Citrus bioflavonoid | 2.44 |
| Seaweed flour | 19.56 |
| Fruit powder rosehips | 2.44 |

3. Orally-ingestible capsule comprising a complex anti-oxidant preparation according to claim 1 or 2.

4. Chewable tablet comprising an anti-oxidant preparation according to claim 1 or 2.

5. Chewable tablet according to claim 4 comprising a breath-freshener.

6. Package having the dimensions of a cigarette package for vending machines, comprising products according to claim 3 to 5.

7. Product-supplying vending machine, comprising multiple separated, selectable goods shafts having packages stacked therein, wherein at least one of the goods shafts comprises cigarette packages and at least one other goods shaft comprises packages according to claim 6.

## Revendications

1. Préparation antioxydante complexe contenant :
de la niacine (B3), de la vitamine C, du bêta-carotène, de la vitamine E, des bioflavonoïdes d'agrumes, de l'extrait de peaux de raisin, de la L-cystine, de l'acide folique, de la poudre d'algues de mer et un système de protection enzymatique avec un composé de zinc, de sélénium, de cuivre et/ou de manganèse.

2. Préparation antioxydante contenant la composition relative suivante (pourcentages en poids), dans laquelle les chiffres en pour-cent peuvent varier respectivement de ± 20% de leur valeur :
| Matière première | Composition % en poids |
|---|---|
| Vitamine C (acide ascorbique) | 29,34 |
| Bêta-carotène à 10% | 7,33 |
| Acétate de vitamine E à 50% | 13,13 |
| Acide folique | 0,54 |
| Amide d'acide nicotinique | 4,40 |
| L-Cystine | 12,22 |
| Levure de sélénium à 0,2% | 6,11 |
| Levure de zinc à 10% | 0,05 |
| Extrait de peaux de raisin | 2,44 |
| Bioflavonoïde d'agrumes | 2,44 |
| Farine d'algues de mer | 19,56 |
| Poudre de cynorrhodons | 2,44 |

3. Capsule pour ingestion orale, contenant une préparation antioxydante complexe selon la revendication 1 ou 2.

4. Comprimé à mâcher, contenant une préparation antioxydante selon la revendication 1 ou 2.

5. Comprimé à mâcher selon la revendication 4, contenant un rafraîchisseur d'haleine.

6. Boîte aux dimensions d'un paquet de cigarettes pour machines automatiques, contenant des produits selon l'une des revendications 3 à 5.

7. Distributeur automatique de produits, contenant plusieurs compartiments d'articles sélectionnables séparément avec des boîtes qui y sont empilées, dans lequel au moins l'un des compartiments d'articles contient des paquets de cigarettes et au moins un autre des compartiments d'articles contient des boîtes selon la revendication 6.
